# EUROPEAN PATENT APPLICATION

(11) **EP 1 495 672 A1**
(43) Date of publication of application: **12.01.2005**
(21) Application number: 02707137.2
(22) Date of filing: 22.03.2002
(51) Int. Cl.: A01H 5/00, C12N 15/09

(54) **FUNCTIONAL PLANT, PROMOTER TO BE USED FOR PRODUCING THE FUNCTIONAL PLANT AND METHOD OF USING THE SAME**

(71) Applicant: Incorporated Administrative Agency National Agriculture Organization and Bio-Oriented Research, Tsukuba-shi, Ibaraki 305-0856 (JP)
(72) Inventor: OTSUKI, Hiroshi, Joetsu-shi, Niigata 943-0835 (JP); OHSHIMA, Masahiro, Joetsu-shi, Niigata 943-0154 (JP)
(74) Representative: Williams, Aylsa
(86) International application number: PCT/JP2002/002817
(87) International publication number: WO 2003/079769

(57) **Abstract**

It is intended to construct a system for arbitrarily expressing a desired gene at a desired site in a plant. This object can be solved by providing a plant containing at least one promoter allowing a specific level of expression and a gene ligated to this promoter in an operable manner wherein the specificity of the promoter is determined based on the expression frequency of a gene containing the promoter in a cDNA database involving the promoter-containing gene. As a cDNA database, use may be made of those published in the genome project.

SEQUENCE LISTING

<110> National Agricultural Research Organization

<120> Functional plants, promoter used to create the same,
      and the use thereof

<130> NG001PCT

<140>
<141>

<160> 12

<170> PatentIn Ver. 2.1

<210> 1
<211> 1705
<212> DNA
<213> Oryza sativa

<400> 1
atcattaggc caaaagatcc cttattcaca acagtccata agaaattgct ttctccacag 60
tacacaccat atttggataa ttgcattgga gcaatagatg gtactcacat ccaagttgtg 120
gtgccaaatt cagctgctgt tcaacatagg aatagacata aggagaagag tcagaatgtt 180
atgtttgtct gtgactttga tatgagattt actttcgtgc ttgctggctg gcctggttcg 240
gttcatgaca tgagggtatt caatgatgca caaactagat ttagtgccaa gtttccaaag 300
ccacctccag gaaagtttta tctcgtagac tcgggatacc caaataggct gggttatcta 360
gcaccataca agggtataac atatcattcc aagagtacaa cgaaagcaca ttgccaaggg 420
gaagaaggga gcactttaat tactgccatt attcgtgtcg gaatgtcata gagaggtcat 480
ttggggtctt gaagaacaag tggaggattt tgtttagttt acctagctac tcgcaggaaa 540
aacaaagcag aattatccat gcatgcatag cacttcataa tttcattaga gacagtcaaa 600
tggctaatac agagttcgac aattgtgacc atgatgaaaa ttatgatcca ttgggtggaa 660

cctctgctcc atctagtgaa ccaacaaacg atttggactc gcgtgtcatg aaccaatttc 720
gggactgggt tgccgatgga ttgtggtctt tgaaaggaat gtaatgaatg gttcaaattt 780
attgtgaatt gtatttggtt taacttgtca attgaaccat gagtgcttgt aatttgtaac 840
attgaaactt ctgtgatgta tttcaacaac ttgagatgtg tatgagcttc tttgaatttc 900
tgtgattagg ccgtgatatt gatgatgaaa atgatgtggg ggttattgct tgtgattcgg 960
cttgctgaag gtgtgtattt gtccaaatta tgatggatta aaacatgtga tgttggggtg 1020
aaacattgat ttggcttgca tttggttgtg ctgtcatgca gcaacaatgg ttttgcttgt 1080
aaagtgctag cagcggcagc aaacagcatg ctgcagcaag gggcatggtg cagtccactt 1140
tgtatggttt tagggggtaa tatggtcatt gtgcagcgca tttaataaac tttagtccct 1200
tttagtccct ataaccaaac agttatggac taaaatgatt agtcctaaga ctaaaataag 1260
tccctaggac ttatggatcc aaacaccacc atagtagaag ctatgggcgg gggcggcagt 1320
ccgggtggca gcatcttcga tagagtggca gccgtccacc ggccgcttca tcgcgcgtgg 1380
cggcagtcca cccagtatgc cgtcgtgggt aaccaatcgg catcgagacc tatccgcggg 1440
acgaagccaa ccgttcgaat ggtgtgcgcg cgtgcatgca tgcgcgcggt ctcagttaat 1500
gcattgcctc tcccgagtca agtggcgtgc tatgtttgca gaggcaaaca tgcatgagca 1560
gatggcctgg tgaacttata tactcccggc aggcaagtga agtgcatgcc tgtatagctt 1620
gagcaagtat ttggcagttt gctgccaact gctaccctat ttcaaatcta ggtaagatct 1680
agctgcaggt tatacagaga aggca 1705

<210> 2
<211> 1543
<212> DNA
<213> Oryza sativa

<400> 2
gatccaaggc atcattccca actacagaag tgcgaggaac atatgttcca cccttccatg 60
taaattcttg catgaacttg gaaaataaaa tgttcgtttt agaatgtata atccctgtcc 120
tacgcaaatt tataactaaa gctaactgtt cattgaatct ttcaggttga gctatatcgc 180
aacgatcagc accgcttcaa gattgttgtt gatggtgaga gcgaagttga cttgatggtg 240
caaactcggc acatgcgaga cgtcatcatt ctggtgatta gaggtcttgc tcaaaagttc 300
aacagcacat ctctcaactc actcctcaag atagaagcat gaagatcgag ttgccaacct 360
gatataactt acagtatgaa accgattaga aaaggttgtt cagtatcgat tcaagttgtt 420

tagtttggag gatgtgtaat atttatagga tttggagcgt ggcagctttt gtgtatgggt 480
gtggttgagt gtgttaaagt tttctttttt ctcaaatcag gagcgggttg ctcttctgac 540
cctggttttc catactcacg aaatgtacat aggaaaggga gggttggtga tatggtgttg 600
cattaatata caagttgtgt atgcagttct catattaggc tatatgattg attgggaaga 660
ttttctgttt tttctggctc gtcatcgttg gtttcatata caaaattcga aatggaaata 720
tgtacgagtg atggttattg aagtgtccat tctattttaa ttctttgcgc gttgtgaggt 780
aatctcatcg tggtgtaggc ttacagcagt aagatgcgtc gccaccgttg tttcacattg 840
caaaatccat ttctgcaatt gcaagcaatg ggcttcaagt tcttgattat ttcagttcat 900
gactcggcat agcaccagca aaggagcaac tggtaaagtg gcacatccac ttcagtgtcg 960
atgcaagcaa ctcattccag cctacaggaa cagtaaatcc aggttcattt actcattttt 1020
ctgtggttga aaaataccaa gatttccatt tttcttactg ctccacataa ttcctatttc 1080
ccattctctt gcacttattc cagcctacag ggacagtaaa tccaggttca tttactcatt 1140
tttctgcggt tgaaaaaata ccaagatttc catttttttt actgctccac ataattcctc 1200
tttcccattc tcttgctcac tatatatgcc tgtcaatttt tttgacagaa catgcatcaa 1260
gtcaccacca acaacatttg cagctgcatc caagaacatt gcatgcagag gtgagcatct 1320
tatccacatt ggcaggctct aaccaggaga tgccacatca gcagcaagga tctgtaacct 1380
atccatgagt ggccactgca gtccacaagc tccccttgtg gctcccattc cacatctcct 1440
ccaagaatta aagcccaatg catctccatg gtggcactgg acattatttt ttttcctcga 1500
aactcagctg caaggagaaa ggagaaggta gggtttccag ccc 1543

<210> 3
<211> 661
<212> DNA
<213> Oryza sativa

<400> 3
ctgaagcatc gatgtttccc tgctctcatt tatctttctt ccatgtaagc aaatttgtct 60
aggtgacacc agagagagac ggtaaataat agtaaataat agcctttgta cattccctta 120
tgtagttgtg cagtctattt ttggtgtgcg agttcggagt tcgaagtgtt ctgaagaatc 180
tcttcttgta atttctagaa atggcattga ggaaattacc agtcttggct taaaaacaag 240
tctgtctcgt cattcaccgt gcttatttag gacttgttca actcctgtag aaattctcga 300
aaattcctcg actttaaatg gagccttacc attttttagc agatgcaaca actattatac 360

tcctacctga tgactgatga ggactgtgca gcgatcgata tgtgcatgtg gccatgtccg 420
aagctaatta aaggacggaa ggaccacaag ttggtgtata ataatggaga gggcattgaa 480
tcattgtttc aatatcccca tcagataata ttcaataact cctatggtga cgtcaagcat 540
caatacatga aggttacttg ttaccaccat cagacaagca tataattgat tgctctgatc 600
aagcgatagc aaaacaaaat acaaaccaga acagtctcaa agctcgccca agcagagaag 660
a 661

<210> 4
<211> 48
<212> DNA
<213> Artificial Sequence

<220>
<223> Description of Artificial Sequence: primer

<400> 4
gtaatacgac tcactatagg gcacgcgtgg tcgacggccc gggctggt 48

<210> 5
<211> 28
<212> DNA
<213> Artificial Sequence

<220>
<223> Description of Artificial Sequence:primer

<400> 5
atgcaagctt tcgacggccc gggctggt 28

<210> 6

<211> 31
<212> DNA
<213> Artificial Sequence

<220>
<223> Description of Artificial Sequence:primer

<400> 6
gcatggatcc tgccttctct gtataacctg c                                31

<210> 7
<211> 1735
<212> DNA
<213> Artificial Sequence

<220>
<223> Description of Artificial Sequence:promoter
      cassette

<400> 7
aagctttcga cggcccgggc tggtatcatt aggccaaaag atcccttatt cacaacagtc 60
cataagaaat tgctttctcc acagtacaca ccatatttgg ataattgcat tggagcaata 120
gatggtactc acatccaagt tgtggtgcca aattcagctg ctgttcaaca taggaataga 180
cataaggaga agagtcagaa tgttatgttt gtctgtgact ttgatatgag atttactttc 240
gtgcttgctg gctggcctgg ttcggttcat gacatgaggg tattcaatga tgcacaaact 300
agatttagtg ccaagtttcc aaagccacct ccaggaaagt tttatctcgt agactcggga 360
tacccaaata ggctgggtta tctagcacca tacaagggta taacatatca ttccaagagt 420
acaacgaaag cacattgcca aggggaagaa gggagcactt taattactgc cattattcgt 480
gtcggaatgt catagagagg tcatttgggg tcttgaagaa caagtggagg attttgttta 540
gtttacctag ctactcgcag gaaaaacaaa gcagaattat ccatgcatgc atagcacttc 600
ataatttcat tagagacagt caaatggcta atacagagtt cgacaattgt gaccatgatg 660
aaaattatga tccattgggt ggaacctctg ctccatctag tgaaccaaca aacgatttgg 720

actcgcgtgt catgaaccaa tttcgggact gggttgccga tggattgtgg tctttgaaag 780
gaatgtaatg aatggttcaa atttattgtg aattgtattt ggtttaactt gtcaattgaa 840
ccatgagtgc ttgtaatttg taacattgaa acttctgtga tgtatttcaa caacttgaga 900
tgtgtatgag cttctttgaa tttctgtgat taggccgtga tattgatgat gaaaatgatg 960
tgggggttat tgcttgtgat tcggcttgct gaaggtgtgt atttgtccaa attatgatgg 1020
attaaaacat gtgatgttgg ggtgaaacat tgatttggct tgcatttggt tgtgctgtca 1080
tgcagcaaca atggttttgc ttgtaaagtg ctagcagcgg cagcaaacag catgctgcag 1140
caaggggcat ggtgcagtcc actttgtatg gttttagggg gtaatatggt cattgtgcag 1200
cgcatttaat aaactttagt cccttttagt ccctataacc aaacagttat ggactaaaat 1260
gattagtcct aagactaaaa taagtcccta ggacttatgg atccaaacac caccatagta 1320
gaagctatgg gcgggggcgg cagtccgggt ggcagcatct tcgatagagt ggcagccgtc 1380
caccggccgc ttcatcgcgc gtggcggcag tccacccagt atgccgtcgt gggtaaccaa 1440
tcggcatcga gacctatccg cgggacgaag ccaaccgttc gaatggtgtg cgcgcgtgca 1500
tgcatgcgcg cggtctcagt taatgcattg cctctcccga gtcaagtggc gtgctatgtt 1560
tgcagaggca aacatgcatg agcagatggc ctggtgaact tatatactcc cggcaggcaa 1620
gtgaagtgca tgcctgtata gcttgagcaa gtatttggca gtttgctgcc aactgctacc 1680
ctatttcaaa tctaggtaag atctagctgc aggttataca gagaaggcag gatcc 1735

<210> 8
<211> 30
<212> DNA
<213> Artificial Sequence

<220>
<223> Description of Artificial Sequence:primer

<400> 8
atgcaagctt gatccaaggc atcattccca 30

<210> 9
<211> 30

<212> DNA
<213> Artificial Sequence

<220>
<223> Description of Artificial Sequence:primer

<400> 9
gcatggatcc ggctggagcc taccttctcc                                        30

<210> 10
<211> 1555
<212> DNA
<213> Artificial Sequence

<220>
<223> Description of Artificial Sequence:promoter
      cassette

<400> 10
aagcttgatc caaggcatca ttcccaacta cagaagtgcg aggaacatat gttccaccct 60
tccatgtaaa ttcttgcatg aacttggaaa ataaaatgtt cgttttagaa tgtataatcc 120
ctgtcctacg caaatttata actaaagcta actgttcatt gaatctttca ggttgagcta 180
tatcgcaacg atcagcaccg cttcaagatt gttgttgatg gtgagagcga agttgacttg 240
atggtgcaaa ctcggcacat gcgagacgtc atcattctgg tgattagagg tcttgctcaa 300
aagttcaaca gcacatctct caactcactc ctcaagatag aagcatgaag atcgagttgc 360
caacctgata taacttacag tatgaaaccg attagaaaag gttgttcagt atcgattcaa 420
gttgtttagt ttggaggatg tgtaatattt ataggatttg gagcgtggca gcttttgtgt 480
atgggtgtgg ttgagtgtgt taaagttttt ctttttctca aatcaggagc gggttgctct 540
tctgaccctg gttttccata ctcacgaaat gtacatagga aagggagggt tggtgatatg 600
gtgttgcatt aatatacaag ttgtgtatgc agttctcata ttaggctata tgattgattg 660
ggaagatttt ctgttttttc tggctcgtca tcgttggttt catatacaaa attcgaaatg 720
gaaatatgta cgagtgatgg ttattgaagt gtccattcta ttttaattct ttgcgcgttg 780

tgaggtaatc tcatcgtggt gtaggcttac agcagtaaga tgcgtcgcca ccgttgtttc 840
acattgcaaa atccatttct gcaattgcaa gcaatgggct tcaagttctt gattatttca 900
gttcatgact cggcatagca ccagcaaagg agcaactggt aaagtggcac atccacttca 960
gtgtcgatgc aagcaactca ttccagccta caggaacagt aaatccaggt tcatttactc 1020
atttttctgt ggttgaaaaa taccaagatt tccatttttc ttactgctcc acataattcc 1080
tatttcccat tctcttgcac ttattccagc ctacagggac agtaaatcca ggttcattta 1140
ctcatttttc tgcggttgaa aaaataccaa gatttccatt ttttttactg ctccacataa 1200
ttcctctttc ccattctctt gctcactata tatgcctgtc aatttttttg acagaacatg 1260
catcaagtca ccaccaacaa catttgcagc tgcatccaag aacattgcat gcagaggtga 1320
gcatcttatc cacattggca ggctctaacc aggagatgcc acatcagcag caaggatctg 1380
taacctatcc atgagtggcc actgcagtcc acaagctccc cttgtggctc ccattccaca 1440
tctcctccaa gaattaaagc ccaatgcatc tccatggtgg cactggacat tatttttttt 1500
cctcgaaact cagctgcaag gagaaaggag aaggtagggt ttccagcccg gatcc 1555

<210> 11
<211> 28
<212> DNA
<213> Artificial Sequence

<220>
<223> Description of Artificial Sequence:primer

<400> 11
gcatggatcc tcttctctgc ttgggcga 28

<210> 12
<211> 691
<212> DNA
<213> Artificial Sequence

<220>

<223> Description of Artificial Sequence:promoter
      cassette

<400> 12
aagctttcga cggcccgggc tggtctgaag catcgatgtt tccctgctct catttatctt 60
tcttccatgt aagcaaattt gtctaggtga caccagagag agacggtaaa taatagtaaa 120
taatagcctt tgtacattcc cttatgtagt tgtgcagtct atttttggtg tgcgagttcg 180
gagttcgaag tgttctgaag aatctcttct tgtaatttct agaaatggca ttgaggaaat 240
taccagtctt ggcttaaaaa caagtctgtc tcgtcattca ccgtgcttat ttaggacttg 300
ttcaactcct gtagaaattc tcgaaaattc ctcgacttta aatggagcct taccattttt 360
tagcagatgc aacaactatt atactcctac ctgatgactg atgaggactg tgcagcgatc 420
gatatgtgca tgtggccatg tccgaagcta attaaaggac ggaaggacca caagttggtg 480
tataataatg gagagggcat tgaatcattg tttcaatatc cccatcagat aatattcaat 540
aactcctatg gtgacgtcaa gcatcaatac atgaaggtta cttgttacca ccatcagaca 600
agcatataat tgattgctct gatcaagcga tagcaaaaca aaatacaaac cagaacagtc 660
tcaaagctcg cccaagcaga gaagaggatc c 691

## Description

### TECHNICAL FIELD

The present invention relates to functional plants, promoters used to create such functional plants, and the use thereof. Specifically, the present invention relates to a method for producing a functional plant by obtaining a specific promoter obtained by the use of a cDNA library.

### BACKGROUND ART

In the field of genetic engineering, a variety of methods are being developed for producing a transgenic plant. In order to specifically express a particular gene in a transgenic plant, it is necessary to introduce a mixture in which the particular gene is operably linked to a genetic element such as a promoter for promoting the expression thereof, into a plant by transformation or the like.
A promoter regulating genetic expression in plants is an essential element for plant genetic engineering. There are a variety of promoters useful for expressing a gene selected in plants (BENFEY P N; REN L; CHUA N-H , EMBO J , 9 (6). 1990. 1685-1696, 1990) . The identification of a promoter region can be made based on the well known technology in the art. Briefly, candidate sequences in a promoter region and a reporter gene (e.g. GUS gene) are operably linked to form an expression cassette. An appropriate plant cell is transformed with such an expression cassette and such a transformed cell is regenerated into a plant body. The expression of the reporter gene in the transformed plant is detected using an appropriate detection system (e.g. dye staining). Based on the result of such detection, the promoter region and the expression properties thereof can be confirmed.

In order to express a gene in a cell (e.g. a plant cell), such a gene should generally be operably linked to a promoter which is recognized by an enzyme in the cell. a region present close to the 5' noncoding region of a gene (that is, a region close to the 5' end of the coding region) usually initiates the transcription of a gene to produce a mRNA transcript. Subsequently, the mRNA is translated in a ribosome of the cell to obtain a polypeptide encoded thereby.

A promoter typically comprises about 500-1500 bases and may provide modified expression of a gene under the control thereof. Expression of an exogenous gene or a selected sequence of a gene in a transgenic plant typically uses a constitutive promoter, which is a promoter that is continuously and globally inducing expression of a gene product in most of the tissues of a plant.

Viral promoters are used to express a selected gene in a plant. Examples of a known viral gene having such a promoter include those found in the Caulimovirus family (a group of a double-stranded DNA virus), the Cauliflower mosaic virus (CaMV) 35S promoter (Balazs et al., 1982; Guilley et al., 1982; Odell et al.,1985; Odell et al.,1987; Odell et al., 1988; Tommerup et al.,1990; Jefferson et al.,1987a; Jefferson, 1987b), the CaMV 19S promoter (Fraley et al., 1994); and the figwort mosaic virus (FMV) promoter (Rogers, 1995).

Promoters such as a promoter derived from Agrobacterium is identified and isolated from a bacterial source which is used for regulating gene expression in a plant. Such promoters include: those derived from an Agrobacterium T-DNA nopaline synthase gene; as well as nopaline synthase (nos) promoter (Rogers, 1991), octopine synthase (ocs) promoter (Leisner and Gelvin, 1988) and mannopine synthase (mas) promoter and the like.

CaMV 35 S promoter has a number of disadvantages among high efficient expression systems. Constitutively high expression, that is to say, a large amount of a gene product is continuously and globally produced in most of the tissues, resulting in the unnecessary loss of metabolites in the tissues which are limited in the plant body, thereby resulting in the reduction of shoot and yield. Consumers are highly concerned by the fact that a portion of a gene product is accumulated in an available site by constitutively high expression of the gene product. Further, constitutively high expression may result in the induction of a silencing mechanism in the plant body.

In addition, few plant promoters (a promoter derived from a plant source) are known which are effective for providing such constitutive expression. A rare number of such examples include: hsp80 which is a heat shock protein from Cauliflower (Brunk and Wilson,1993), and tomato ubiquitin promoter (Picton et al.,1993). These promoters can be used for inducing consitutive expression of an exogenous nucleic acid sequence in a plant tissue to be transformed. However, like the CaMV 35S promoter, these promoters globally and continuously produce a large amount of a gene product, resulting in the unnecessary waste of limited metabolic products inside the plant body, and raising a number of problems such as the reduction in the amount of product or a reduction in yield. Accumulation of constitutively high expression of gene products in available sites is becoming concern with consumers, and constitutively high expression in the plant body may lead to induction of silencing machinery.

Therefore, it is difficult to produce a transgenic plant realizing the desired functional expression using conventional promoters.

### DISCLOSURE OF THE INVENTION

Therefore, it is an object of the invention to produce a functional plant, and particularly, it is an object of the present invention to construct a system for expressing a desired gene in a desired site of a plant in an arbitary manner.

The present invention has solved the above-identified problems by recognizing expression frequency in a cDNA library data base, isolating a promoter comprised in the genes having the desired expression frequency, utilizing such promoters together with a desired gene, and producing a functional plant expressing the desired gene product at the desired site. Therefore, the present invention provides the following:
1. A plant comprising at least one promoter promoting a specific level of expression and a gene operably linked to the promoter, wherein the specificity of the promoter is determined based on the expression frequency of the gene including the promoter in a cDNA database in which the gene including the promoter is included.
2. The plant according to item 1 wherein the gene is constitutively expressed.
3. The plant according to item 1 wherein the gene is specifically expressed in leaves.
4. The plant according to item 1 wherein the gene is specifically expressed in callus.
5. The plant according to item 1 wherein the plant is rice.
6. The plant according to item 1 wherein the gene is a disease resistance gene or an insect resistance gene.
7. The plant according to item 1 wherein the gene is a gene selected from the group consisting of a vitamin synthesizing gene, a carbohydrate synthesizing gene, a lipid synthesizing gene, a polyketide synthesizing gene, a photosynthesizing system gene, a functional component synthesizing gene and a transcription factor.
8. A promoter promoting a specific level of expression, wherein the specificity of the promoter is determined based on the expression frequency of the gene including the promoter in a cDNA database in which the gene including the promoter is included.
9. The promoter according to item 8 which does not drive the expression of a gene in fruit.
10. The promoter according to item 8 which specifically drives the expression of a gene in leaves.
11. The promoter according to item 8 which specifically drives the expression of a gene in callus.
12. An expression cassette comprising a promoter wherein the specificity of the promoter is determined based on the expression frequency of the gene including the promoter in a cDNA database in which the gene including the promoter is included.
13. The expression cassette according to item 12 which promotes the constitutive expression of a gene.
14. The expression cassette according to item 12 which specifically drives the expression of a gene in leaves.
15. The expression cassette according to item 12 which specifically drives the specific expression of a gene in callus.
16. An available site of a plant which is obtained from a plant comprising at least one promoter promoting a specific expression and a gene operably linked to the promoter, wherein the specificity of the promoter is determined based on the expression frequency of the gene including the promoter in a cDNA database in which the gene including the promoter is included.
17. The available site according to item 16 which is a leaf, a stem or a fruit.
18. A method for producing a desired gene product, comprising the steps of:
   determining the specificity of a promoter wherein the specificity of the promoter is determined based on the expression frequency of the gene including the promoter in a cDNA database in which the gene including the promoter is included;
   producing a linked mixture by operably linking the promoter and a nucleic acid molecule encoding the desired gene product;
   introducing the linked mixture into a plant;
   growing the plant; and
   collecting the desired gene product in the plant.
19. A method for producing a plant specifically expressing a desired gene product, comprising the steps of:
   determining the specificity of a promoter wherein the specificity of the promoter is determined based on the expression frequency of the gene including the promoter in a cDNA database in which the gene including the promoter is included;
   producing a linked mixture by operably linking the promoter and a nucleic acid molecule encoding the desired gene product;
   introducing the linked mixture into a plant; and
   growing the plant.
20. The method according to item 19 wherein the specificity of the promoter is determined by the analysis of a DNA chip.
21. A gene product which is obtained from the plant obtained by the method according to item 19.

### BRIEF DESCRIPTION OF DRAWINGS

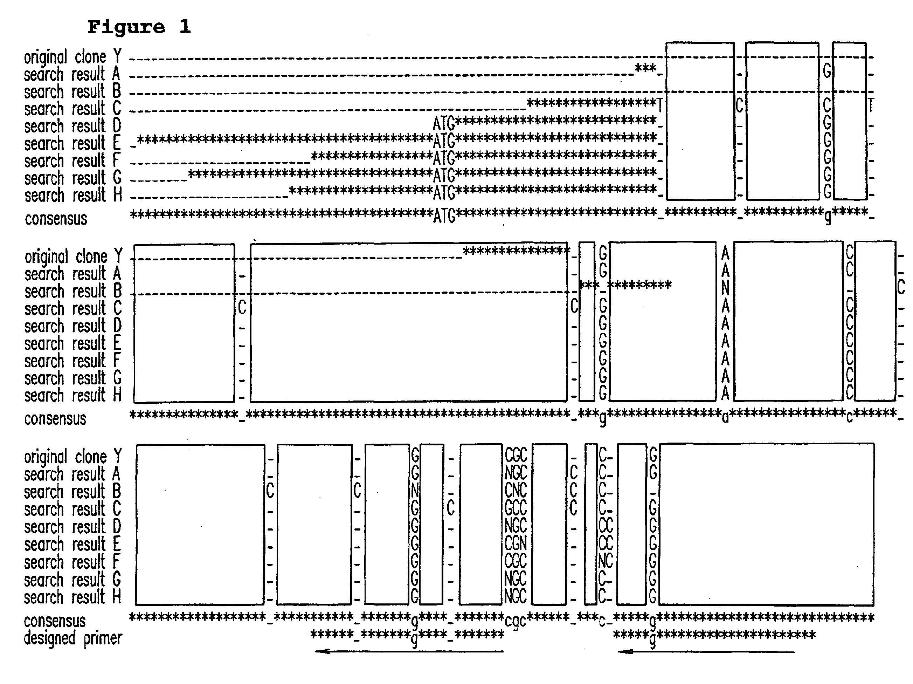

**Figure 1** shows an exemplary sequences which are considered to belong to the same group based on the result of a homology search. Most of the EST sequence data used were obtained from a DNA sequencer. Except for errors from DNA sequencers, a number of clones obtained from the homology search were compared to design primers for primer walking. Portions circled by boxes and the symbol * show the same sequence in all clones. The symbol - shows a gap. "Original" clone shows a reference clone of the database. "Search result" shows the result of the homology search. "Consensus" shows consensus sequences. "Designed primer" shows a designed primer.

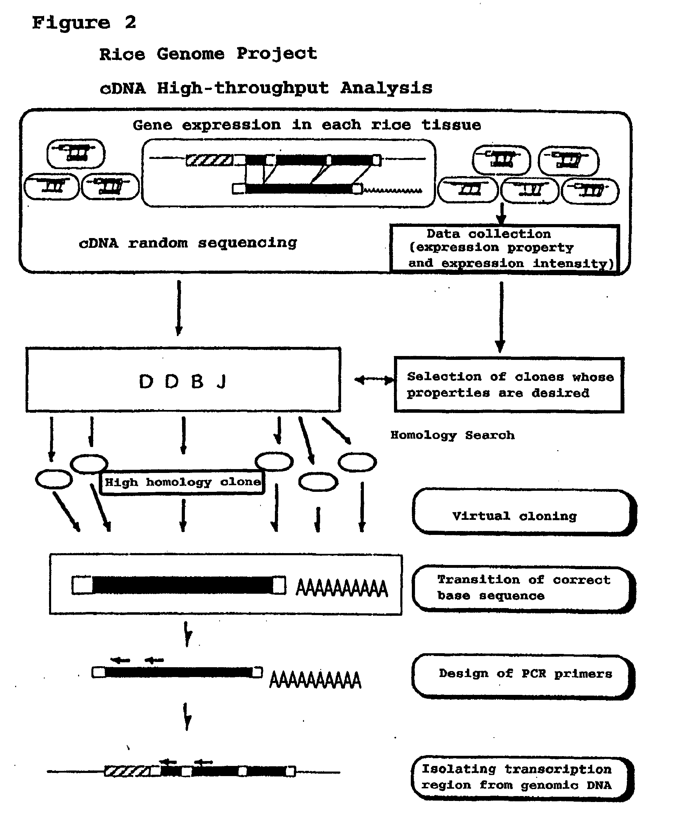

**Figure 2** shows an overview of a method for isolating transcription controlling regions.

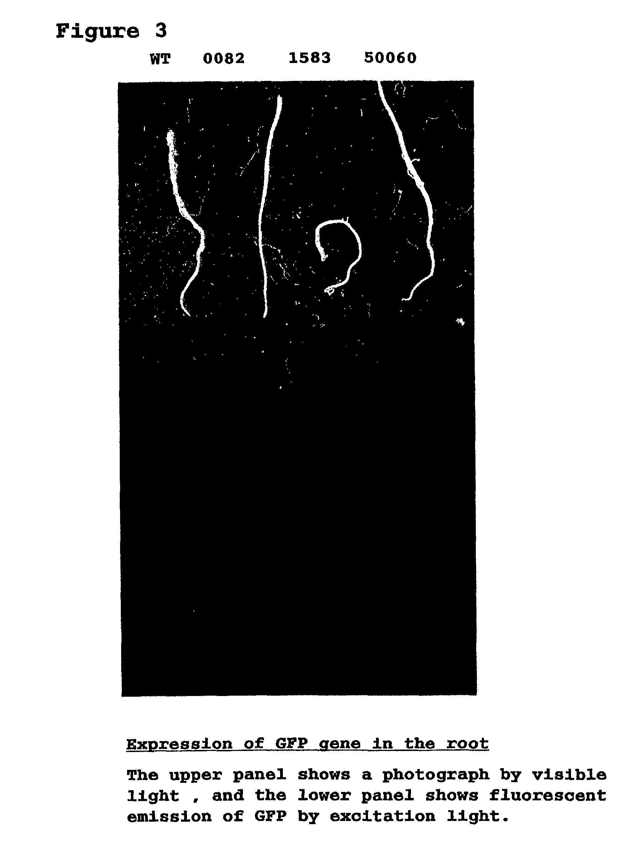

**Figure 3** shows a drawing showing the expression of GFP gene in the root. The upper panel shows a photograph by visible light, and the lower panel shows fluorescent emission of GFP by excitation light. WT shows wild-type, 0082 shows a clone belonging to EB0082_2, 1583 shows a clone belonging to RA1583_1, and 50060 shows a clone CF0060_1.

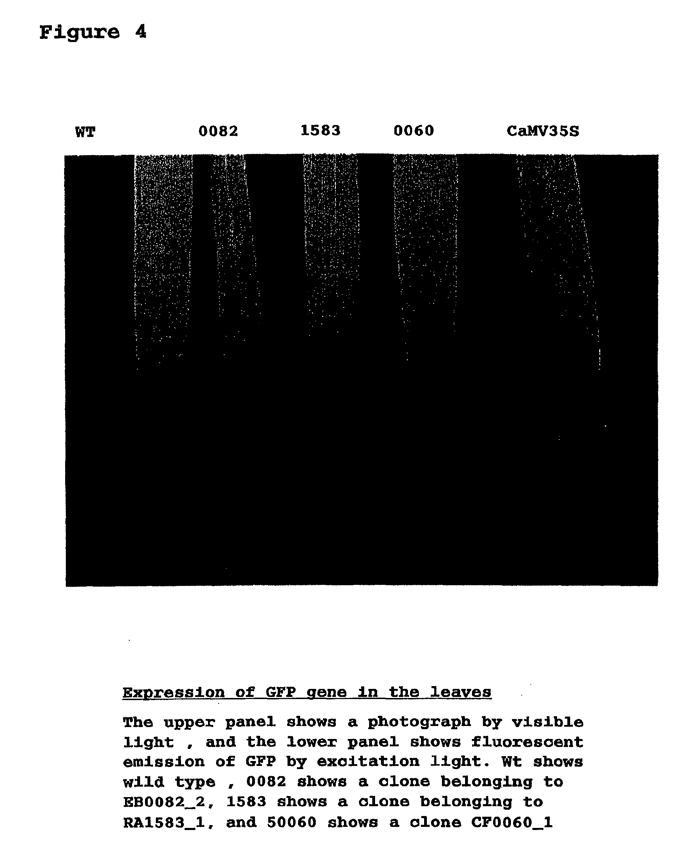

**Figure 4** shows expression of GFP gene in the leaves. The upper panel shows a photograph by visible light, and the lower panel shows fluorescent emission of GFP by excitation light. WT shows wild-type, 0082 shows a clone belonging to EB0082_2, 1583 shows a clone belonging to RA1583_1, and 50060 shows a clone CF0060_1.

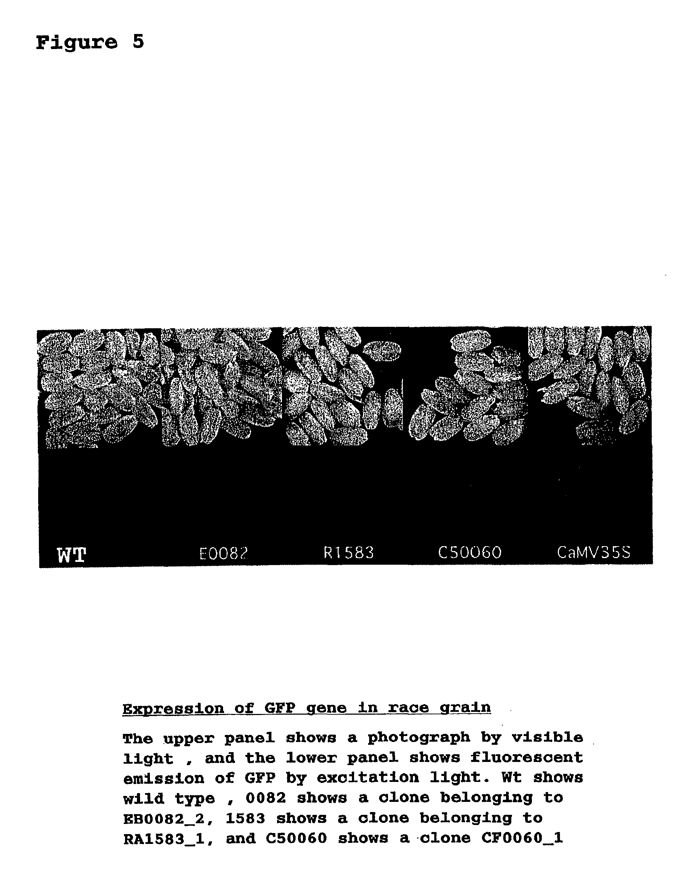

**Figure 5** shows expression of GFP gene in rice grain. The upper panel shows a photograph by visible light, and the lower panel shows fluorescent emission of GFP by excitation light. WT shows wild-type, 0082 shows a clone belonging to EB0082_2, 1583 shows a clone belonging to RA1583_1, and 50060 shows a clone CF0060_1.

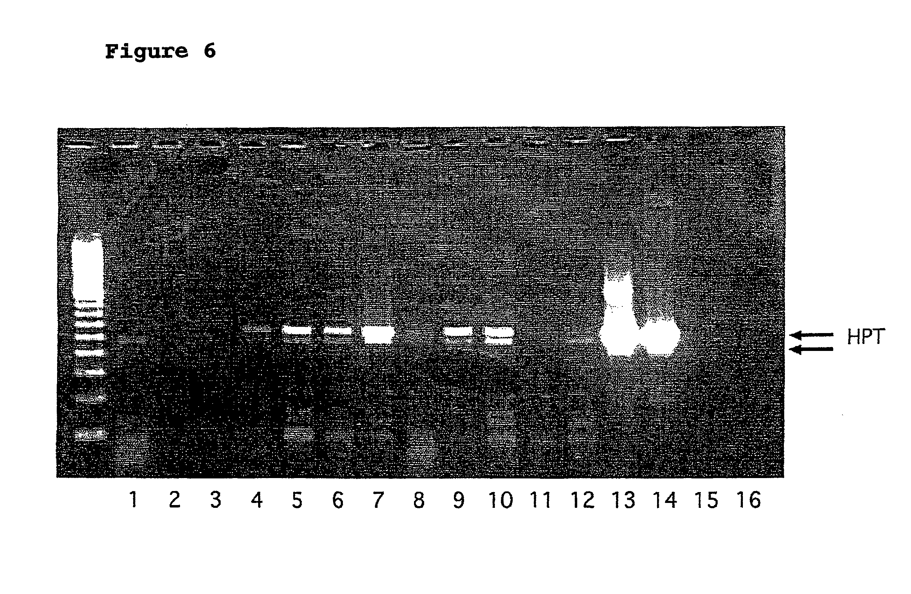

**Figure 6** shows detection of the HPT gene (the site shown by the upper arrow) by conducting PCR using DNA extracted from grown callus as a template. The left extreme shows the size marker, lanes 1 to 3 show the result of PCR, as a template, using DNA extracted from calli without the gene introduced, lanes 4 to 6 show vectors in which HPT gene is driven by the CaMV35S promoter, lanes 7 to 9 show a vector in which the HPT gene is driven by the RA1581_1 derived promoter which is derived from a clone belonging to RA1581_1, lanes 10 to 12 show a vector in which the HPT gene is driven by the CG0060_1 derived promoter which is derived from a clone belonging to CG0060_1, lanes 13 and 14 show the HPT gene fragment and the pTH1 plasmid as positive control, lane 15 shows extraction buffer used for DNA extraction as negative control, lane 16 shows sterile water as template. The upper arrow shows the expected size of the product to be amplified of the HPT gene. Lanes 15 and 16 did not show amplified product.

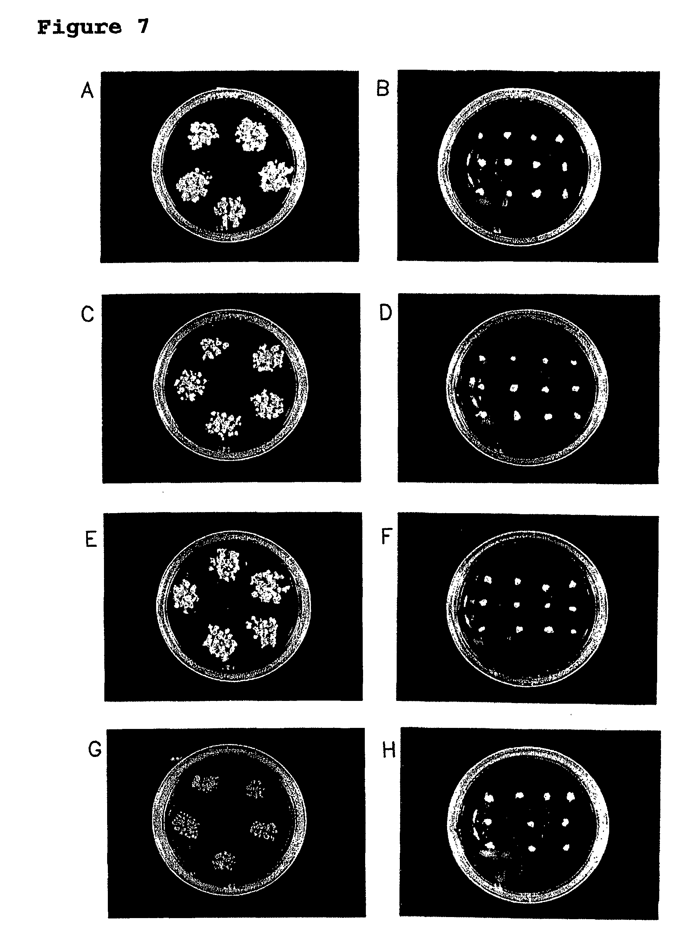

**Figure 7** shows a photograph showing the growth of each callus before and after the addition of hygromycin. A and B show the result of using the promoter region of a clone derived from RA1581_1, C and D show the result using the promoter region of a clone derived from CG0060_1, E and F show the result using a vector having the structure driving the HPT gene by the CaMV35S promoter as positive control, and G and H show the result using wild type. A, C, E and G show the result after the growth in the presence of hygromycin, and B, D, F and H show the result before the growth.

### (Description of Sequence Listing)

SEQ ID NO: 1 is the promoter region of a clone derived from CG0060_1.
SEQ ID NO: 2 is the promoter region of a clone derived from EB0082_2.
SEQ ID NO: 3 is the promoter region of a clone derived from RA1583_1.
SEQ ID NO: 4 is adapter primers for GenomeWalker.
SEQ ID NO: 5 is primers comprising a part of the adapter primers and restriction enzyme recognition sequences.
SEQ ID NO: 6 is primers comprising the sequence before the translation starting point and restriction enzyme recognition sequences.
SEQ ID NO: 7 is a promoter cassette comprising the promoter region of a clone derived from CG0060_1 with restriction enzyme recognition sequences added at both termini.
SEQ ID NO: 8 is primers comprising a part of rice genome sequence and restriction enzyme recognition sequences use in Example 5.
SEQ ID NO: 9 is primers comprising the sequence before the translation starting point and restriction enzyme recognition sequences used in Example 5.
SEQ ID NO: 10 is a promoter cassette comprising the promoter region of a clone derived from EB0082_2 with restriction enzyme recognition sequences added at both termini.
SEQ ID NO: 11 is primers comprising the sequence before the translation starting point and restriction enzyme recognition sequences used in Example 6.
SEQ ID NO: 12 is a promoter cassette comprising the promoter region of a clone derived from RA1583_1 with restriction enzyme recognition sequences added at both termini.

### BEST MODE FOR CARRYING OUT THE INVENTION

Hereinafter, the present invention will be described. It should be understood throughout the present specification that articles for a singular form (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise stated. It should be also understood that the terms as used herein have definitions typically used in the art unless otherwise stated.

As used herein, the term "functional plant" refers to a plant in which a function is significantly (preferably, statistically) changed (for example, added, enhanced, reduced, removed or the like) in comparison to the plant in the naturally occurring state. As used herein the "function" of a plant refers to a physiological action of such a plant.

As used herein, the term "promoter" refers to a region of DNA which determines the initiating site of transcription of a gene and directly regulating the occurrence thereof, and is usually a base sequence of initiating the transcription by binding a RNA polymerase.

As used herein the term "promoter region" refers to a region close to a structural gene sequence and having promoter activity. "Deduced promoter region" refers to a region which is considered to be a region close to a structural gene sequence and having promoter activity. Such deduced promoter regions are regions which are deduced to be base sequences upstream of a gene which are bound by a RNA polymerase when initiating the transcription of a gene and as often located within about 2 kbp upstream of the first exon of deduced protein coding region. Therefore, promoter regions can be deduced by predicting the protein-coding region in the genome base sequences using DNA analysis software. The deduced region may vary depending on structural genes, and may usually be located but not limited to upstream of the structural gene and may be located downstream of the structural gene. Preferably, the deduced promoter region is located within about 2 kbp upstream of the first exon translation stating point.

A nucleic acid comprising at least ten contiguous nucleotide sequence of the promoter sequence of the present invention may have the identical or similar activity to that of the promoter of the present invention. Such an activity may be confirmed by an assay using beta glucuronidase (GUS) gene, luciferase gene or green fluorescence protein (GFP) gene as a reporter gene, or a biochemical or cell histological assay. Such assays are within the well known and routine technology of the art (Maliga et al., Methods in Plant Molecular Biology: A laboratory course. Cold Spring Harbor Laboratory Press (1995); Jefferson, Plant Molec. Biol. Reporter 5: 387 (1987); Ow et al., Science 234: 856 (1986); Sheen et al., Plant J. 8: 777-784(1995)), therefore those skilled in the art can confirm without undue experimentation that a nucleic acid comprising at least ten contiguous nucleotides of the sequence of the present invention will have the identical or similar activity or equal or substantially more than equal to that of the promoter of the present invention. As used herein, in the above-mentioned assay, when determined to have the same promoter activity within the range of detection error, such a promoter is considered to have substantially the same or more promoter activity.

The length of the promoter of the present invention is usually at least 10 nucleotides, however, preferably may be at least 20 nucleotides, at least 30 nucleotides, at least 40 nucleotides, at least 50 nucleotides, at least 60 nucleotides, at least 70 nucleotides, at least 80 nucleotides, at least 90 nucleotides, at least 100 nucleotides, at least 150 nucleotides, at least 200 nucleotides, or at least 300 nucleotides in length.

The promoter of the present invention can be used by linking a conventional promoter (e.g., minimum promoter (a promoter consisting of about 80 base pairs derived from the 35S promoter (Hatton et al., Plant J., 7:859-876 (1995) ; Rouster et al., Plant J., 15: 435-440 (1998) ; Washida et al., Plant Mol. Biol., 40:1-12 (1999)) or the like) thereto. In this case, even the conventional promoter to be linked shows no or little tissue specificity or different specificity, adding the promoter of the present invention or a fragment thereof can result in the promoter having a function of strong tissue specific expression in the root and/or shoot apex (Hatton et al., Plant J., 7:859-876 (1995) ; Rouster et al., Plant J., 15: 435-440 (1998) ; Washida et al., Plant Mol. Biol., 40:1-12 (1999)).

As used herein the term "plant" collectively refers to an organism belonging to the plant kingdom, and typically a flowering plant having the formation of cell wall and assimilation actions by chlorophyll. The "plant" includes both monocotyledonous and dicotyledonous plants. Preferable plants include, for example, monocotyledonous plants including poaceous plants such as wheat, maize, rice, barley, sorghum or the like. Other preferable plants include tobacco, green pepper, aubergine, melon, tomato, sweet potato, cabbage, Welsh onion, broccoli, carrot, cucumber, oranges, Chinese cabbage, lettuce, peach, potato and apple and the like. Preferable plants are not limited to crop plants but also include flowers, trees, grasses, weeds and the like. Unless otherwise specifically indicated, plants will mean any of the plant body, plant organ, plant tissue, plant cell and seed. Examples of plant organs will include the roots, leaves, stems and flowers. Examples of plant cell include callus and suspension culture cells.

As used herein the term "available site" of a plant refers to a part of a plant which an organism can ingest when such an organism ingests such a plant. Therefore, for example, when the organism is a human, and the plant is rice, "available site" can be referred to as "edible part" and such available site will include a rice grain. It can be said that albumen in such a rice grain to be eaten as white or polished rice, corresponds to such an edible part. Rice grain refer to albumen and germ amongst the lemma and palea, germ, albumen, and seed coat which consist the rice grain.

If the organism is a cow, and the plant is rice, then the available site is the whole plant except for the roots. As used herein the "fruit" refers to a part forming a seed developing a fertilized ovule in the organ fruit in which an ovary is developed (in the poaceous plant, particularly refers to caryopsis). As used herein the term "seed" refers to a constant diaspore formed by developing an ovary therein, and consisting of germ, albumen and seed coat.

As used herein, the term "gene" refers to a functional unit of heredity, and usually occupies a particular site (locus) on the chromosomes. A gene usually correctly replicates itself during mitosis and controls the synthesis of different proteins such as an enzyme. A gene, as a functional unit, consists of a discontinuous segment of DNA macromolecules, and the DNA macromolecule comprises a base sequence (A, T, G and C) correctly encoding an amino acid sequence of a particular peptide. A gene is usually described by DNA with respect to the information thereof, but may be set forth by RNA. As described above, a gene is usually located in the chromosomes, and all the chromosomes form a pair except for the male sex chromosomes (X and Y) of a human for example, a gene is usually present as a pair in all the cells except for gametes and this is true of plants. A gene comprises a region encoding a protein (an exon), and an intron existing between the exons, and an expression controlling region (promoter region) usually upstream of the first exon and a downstream region of the protein encoding region.

As used herein, "specific level of expression" of a gene refers to the expression at a different level (preferably high level) at a particular site or period in comparison to the other sites or during a particular periods. Such a specific level of expression can be an expression at a particular site (specific site) only, or can include the expression at sites other than the specific site. Preferably, the specific level of expression refers to the expression at the particular site only.

As used herein the term "operably linked" refers to the location of the expression (operation) of a desired sequence of desire under the control of a particular transcription controlling sequence (for example, a promoter) or translation controlling sequence. In order that a promoter is to be operably linked to a gene, such a promoter is usually located just upstream of the gene, but it is not necessarily located just adjacent thereto. Therefore, in this case, "the promoter is operably linked to the gene" refers to any relative positional relationship so long as the promoter can control the expression of the gene.

As used herein in relation to the expression of a gene in plants, "site specificity" generally refers to the specificity of the expression of the gene in a plant site (for example, root, stem, stock, leaves, flower, seed, germ, embryo, fruit or the like). "Period specificity" refers to the specificity of the expression of the gene depending on the growth step of a plant (for example, the number of the days after starting germination). "Stress response" refers to the change of the expression of a gene upon at least one stress given to a plant.

As used herein, a "cDNA database" refers to a database by synthesizing a cDNA from mRNA derived from a plant tissue, and sequencing a large number of clones. In the rice genome project, a database has been constructed by producing cDNA libraries derived from mRNA extracted from each of benzyl adenine treated callus, gibberellin treated callus, heat shock treated callus, callus in the growth period, young root, young green leaves, young etiolated leaves, flowering ear, and randomly sequencing the base sequence thereof. The expression frequency of genes in a cDNA database is calculated by calculating the ratio of the individual which has actually expressed against the number of the samples actually used. A cDNA database may be a database made based on a database published in the genome project such as rice genome project or the like. The specificity of promoters determined based on the expression frequency, depends on the expression frequency of the gene comprising the promoter in a cDNA database comprising the gene comprising the promoter. Thus, the specicifity of the promoter used in the present invention will be detemined based on the expression frequency of the gene comprising the promoter in a cDNA database.

Thus, based on the description of the present application, those skilled in the art will be able to give a desired specific expression property to a gene of interest, and produce a plant expressing a gene of interest with the desired specific property.

As used herein, the gene introduced is "not expressed" refers to that the gene is not expressed at all or expressed at a level which does not substantially adversely affect the host in which the gene is introduced, including for example, that the gene is expressed below the standard level defined by the governmental authority.

As used herein to "drive" the expression of a gene by an agent refers to that the expression of the gene is promoted by locating the gene under the control of the agent. As used herein, "not to drive" the expression of a gene by an agent refers to that the expression of the gene is not promoted at all or expressed at a level not substantially adversely affecting the host to which the gene is introduced even if the gene is located under the control of the agent. In the case where an agent does not drive the expression of a gene, the gene is expressed under the standard level defined by the governmental authority.

As the promoter of the present invention the sequence similar to the promoter as described can be used. Such a sequence can be selected by determining the homology by using a sequence comparison program such as FASTA, or by using hybridization technology.

As used herein, "stringent conditions" for hybridization refers to conditions under which a complementary strand of nucleotide strand having homology against a target sequence preferentially hybridizes to the target sequence, and a complementary strand of nucleotide strand having no homology does not substantially hybridize thereto. As used herein a "complementary strand" of a nucleic acid sequence refers to a nucleic acid sequence forming a pair based on the hydrogen binding between the bases of a nucleic acid (for example, T against T, C against G). Stringent conditions are sequence-dependent, and will vary depending on a variety of circumstances. The longer the sequence is, the higher will the probability that the sequence will specifically hybridize. Generally, stringent conditions are selected as 5 °C below the thermal melting temperature (Tₘ) for a particular sequence at a defined ionic strength and pH. Tₘ is the temperature at which 50 % of a complementary nucleotide complementary to a target sequence hybridizes to the target sequence in an equilibrium status under a defined ionic strength, pH and nucleic acid sequence. "Stringent conditions" are sequence-dependent, and will vary depending on a variety of circumstantial parameters. Such general guidance for nucleic acid hybridization is found in Tijssen (1993), Laboratory Technniques In Biochemistry And Molecular Biology-Hybridization With Nucleic Acid Probes Part I, Sec. 2 "Overview of principles of hybridization and the strategy of nucleic acid probe assay", Elsevier, New York.

As a general molecular biology technology, those skilled in the art can carry out such technology by referring to Ausubel F.A. et al., Ed. (1988), Current Protocols in Molecular Biology, Wiley, New York, NY ; Sambrook J. et al., (1987) Molecular Cloning: A Laboratory Manual, 2nd Ed., Cold Spring Harbor Laboratory Press, Cold Spring Harbor, NY an the like.

Typically, stringent conditions refer to the salt concentration of about 1.0 M Na⁺ or below, typically refers to the pH of 7.0 to 8.3 and Na⁺ (or other salt) concentration of about 0.01 to 1.0 M, and the temperature is about at least 30 °C for shorter nucleotides (for example, 10 to 50 nucleotides), and at least 60 °C for longer nucleotide (for example, longer than 50 nucleotides). Stringent conditions can also be achieved by the addition of an unstabilizer such as formamide. As a stringent condition used herein, hybridization under 50 % formamide, 1M NaCl, 1% SDS (37 °C) and washing under 0.1 x SSC at 60 °C can be used.

As used herein "stress" refers to an agent inhibiting the proper growth of a plant which may be added to the plant in a physical, chemical or biological manner. Stress includes physical stress (such as light, heat, cold, freezing, ultraviolet, X-ray, cutting, rubbing or the like), chemical stress (such as oxygen stress, chemicals, physiologically active substance or the like), biological stress (such as virus, pathogen (for example, rice blast bacteria) infection or the like), and the like. Thus, the promoter of the present invention includes those promoters which will specifically express the gene to which they are operably linked when exposed to such a stress.

As used herein, that the expression of the promoter of the present invention is "constitutive" refers to a property of expression where the expression is substantially the same in all the tissues of a plant or during either the younger period and the adult period of the growth thereof. Specifically, when analyzing by Northern blotting under conditions similar to the Examples of the present specification, for example, at any points (such as at two or more time points (such as at day 5 and day 15)), when expression is found at the same sites or the site corresponding to the other, such an expression is considered to be constitutive under the definition of the present invention. A constitutive promoter is considered to play a role in maintaining homeostatis of a plant in usual growth circumstances. As used herein , that the expression of the promoter of the present invention is "stress responsive" refers to a property where the amount of expression is changed when at least one stress is given to a plant body. Particularly, a property where the amount of expression is increased refers to "stress inductive", and a property where the amount of expression is decreased refers to "stress reductive". The expression of the "stress reductive property" is based on the fact that an expression is observed in a normal state, therefore, it may overlap the concept of "constitutive" expression. These properties may be determined by analyzing the expression amount by extracting RNA from any portion of a plant and conducting Northern blotting analysis, or quantification of the protein expressed by Western blotting.

As used herein "disease resistance" refers to a property of a plant having capability of reducing the level of pathogenicity. As used herein "insect resistance" refers to a property of a plant having capability of reducing the level of insect damage. As used herein "disease specific" refers to a property where a promoter will have a specificity when a disease occurs. As used herein, "insect damage specific" refers to a property where a promoter will have a specificity when insect damage occurs. As used herein "drug resistant gene" refers to a gene rendering a host drug resistant when the product of the gene is expressed in the host. Such a drug resistance gene is desirably those facilitating the selection of transformed plants, and preferably include the neomycin phosphotransferase II (nptII) gene rendering kanamycin resistance, hygromycin phosphotranferase gene rendering hygromycin resistance.

The promoter of the present invention can be used to modify not only monocotyledonous plants but also other biological organisms such as dicotyledonous plants. This can be readily understood where basically both have similar transcriptional regulation machinery. For example, it has been reported that the zein gene promoter of maize was expressed with the same tissue characteristic in tobacco (Schernthaner et al., EMBO J., 7:1249-1255 (1988)); and the glutelin gene promoter of rice was expressed with the same tissue characteristic in tobacco (Leisy et al., Plant Mol. Biol., 14: 41-50 (1989)). Particularly preferable subject plants include in addition to rice; wheat, maize, barley, sorghum, citrus, Chinese cabbage, lettuce, tobacco, peach, potato, tomato, and apple.

When a gene is herein discussed, "vector" refers to a vector capable of introducing a polynucleotide sequence of interest into a target cell. Such a vector includes a vector containing a promoter capable of self-replication in prokaryotic cells, yeast, animal cells, plant cells, insect cells, individual animals or being incorporated into the chromosome thereof, and positioned at a site suitable for the transcription of a polynucleotide of the present invention. "Recombinant vector" for plant cells includes Ti plasmid, a tobacco mosaic virus vector, and the like. Expression cassette is preferably in a form of a plant expression vector. "Plant expression vector" refers to a nucleic acid sequence in which various regulatory elements as well as a structural gene and a promoter for regulating the expression are operatively linked in a host plant cell. Examples of the regulatory elements include, preferably, terminators, drug-resistant genes, and enhancers. It is well known to those skilled in the art that the types of plant expression vectors and the types of regulatory elements used may vary according to a host cell. The plant expression vectors used in the present invention may further have a T-DNA region. The T-DNA region can improve the efficiency of gene introduction when plants are transformed by, particularly, *Agrobacterium.*

"Terminator" is a sequence which is located downstream of a region encoding a protein of a gene and which is involved in the termination of transcription when DNA is transcribed into mRNA, and the addition of a poly A sequence. It is known that a terminator contributes to the stability of mRNA, and has an influence on the amount of gene expression. Examples of such a terminator include, but are not limited to, a CaMV35S terminator, a terminator for the nopaline synthetase gene (Tnos), and a terminator for the tobacco PR1a gene.

"Enhancer" may be used so as to enhance the expression efficiency of a gene of interest. As such an enhancer, an enhancer region containing an upstream sequence within the CaMV35S promoter is preferable. A plurality of enhancers may be used.

As a vector for use in construction of a plant expression vector, pBI vectors, pUC vectors, or pTRA vectors are preferably used. The pBI and pTRA vectors may be used to introduce a gene of interest into plants via Agrobacterium. pBI binary vectors or intermediate vectors may be preferably used. Examples of such vectors include pBI121, pBI101, pBI101.2, pBI101.3, and the like. These vectors contain a gene of a region (T-region) to be introduced into a plant, and the nptII gene (conferring kanamycin resistance) as a marker gene which is expressed under the control of a plant promoter. With pUC vectors, a gene may be introduced directly into plants. Examples of pUC vectors include pUC18, pUC19, pUC9, and the like. Plant expression vectors may be produced using gene recombinant techniques well known to those skilled in the art.

For the purpose of introduction of a plant expression vector into a plant cell, a method well known to those skilled in the art, such as an indirect method using Agrobacterium, and a method for directly introducing the gene into cells, can be used. As such an indirect method using Agrobacterium, for example, a method of Nagel et al. (Nagel et al. (1990), Microbiol. Lett., 67, 325) may be used. In this method, initially Agrobacterium is transformed with a plant expression vector by electroporation, and then the transformed Agrobacterium is introduced into a plant cell with a method described in Gelvin et al. (Gelvin et al. ed. (1994), Plant Molecular Biology Manual (Kluwer Academic Press Publishers)). As a method for directly introducing a plant expression vector into a cell, an electroporation method (see Shimamoto et al., (1989), Nature, 338: 274-276; and Rhodes et al., (1989), Science, 240: 204-207), a particle gun method (see Christou et al. (1991), Bio/Technology 9: 957-962), and a polyethylene glycol (PEG) method (see Datta et al. (1990), Bio/Technology 8: 736-740) are illustrated. These methods are well known in the art. A method suitable for a plant to be transformed can be appropriately selected by those skilled in the art.

A cell into which a plant expression vector has been introduced is first selected according to drug resistance, such as kanamycin resistance, and the like. Thereafter, the cell may be regenerated to a plant tissue, a plant organ, and/or a plant using a well-known method in the art. Further, seeds may be obtained from the plant. The expression of introduced genes may be detected by a northern blotting method or a PCR method. The expression of proteins which are genetic products may be confirmed by, for example, a western blot method.

Examples of a vector introducing method include any method of introducing DNA into plant cells, for example, transfection, transduction, and transformation using the following: Agrobacterium (Japanese Laid-Open Publication No. 59-140885, Japanese Laid-Open Publication No. 60-70080, and WO94/00977), an electroporation method (Japanese Laid-Open Publication No. 60-251887), a particle gun (gene gun) method (Japanese Patent No. 2606856 and Japanese Patent No. 2517813), or the like.

"Detection" or "qualification" of expression of the promoter of the present invention may be achieved using an appropriate method including, for example, mRNA measurement and immunological measurement methods. Molecular biological measurement methods include, for example, a northern blot method, a dot blot method, a PCR method, and the like. Immunological measurement methods include, for example, an ELISA method, an RIA method, a fluorescent antibody method, a western blot method, an immunohistological staining method, and the like using a microtiter plate. Quantitation methods include an ELISA method, an RIA method, and the like.

"Expression level" refers to of the level of a protein or mRNA of the present invention expressed in a target cell or the like. Such an expression level includes the protein level of the polypeptide of the present invention evaluated by any appropriate method including immunological measurement methods, such as an ELISA method, an RIA method, a fluorescent antibody method, a western blot method, an immunohistological staining method, and the like, using an antibody of the present invention; or the mRNA level of the polypeptide of the present invention evaluated by any appropriate method including biological measurement methods including a northern blot method, a dot blot method, a PCR method, and the like. "Change in an expression level" refers to an increase or decrease in an expression level determined by the protein or mRNA level of the peptide of the present invention, evaluated by any appropriate method including the above-described immunological or biological measurement methods. By observing the absolute or relative value of the expression level, it is possible to determine whether or not a certain promoter acts specifically.

"Transformant" refers to the whole or a part of an organism, such as a cell or the like, produced by transformation. Examples of the transformant include prokaryotic cells, yeast, animal cells, plant cells, insect cells, and the like. The transformant is also called a transformed cell, transformed tissue, a transformed host, or the like, depending on what is transformed.

"Redifferentiation" as used herein refers to a phenomenon that a whole entity is reconstructed from a part of an entity. For example, a plant body is formed from a piece of tissue, such as a cell, a leaf, a root, or the like.

A method of redifferentiating a transformant to a plant body is well known in the art. Such a method is illustrated in Rogers et al., Methods in Enzymology 118: 627-640 (1986); Tabata et al., Plant Cell Physiol., 28: 73-82 (1987); Shaw, Plant Molecular Biology: A practical approach. IRL press (1988); Shimamoto et al., Nature 338: 274 (1989); Maliga et al., Methods in Plant Molecular Biology: A laboratory course. Cold Spring Harbor Laboratory Press (1995); and the like. Therefore, it is possible for those skilled in the art to use the above-described well-known method, depending on a transgenic plant of interest, so as to carry out redifferentiation.

The presence of a desired, modified property of a redifferentiated transformant may be confirmed by performing an appropriate assay depending on the type of the property. For example, the GUS gene as a reporter gene fused with a promoter which is in turn introduced into a plant body to produce a transformant. The transformation can be confirmed by detecting GUS activity by histological staining. In this case, a GFP gene or a luciferase gene, which are fluorescent proteins, can be used as a reporter gene. Any of these genes are used in assays for various promoters. The details of the genes will be described in the examples below. When it is intended to confer resistance against pathogenic bacteria (stress resistance), a model bacterium (e.g., Pseudomonas syringae pv.tabaci) is inoculated into a redifferentiated plant body. In this case, a property change in the redifferentiated plant body may be evaluated by observing the presence or absence of the change by comparing it with a control plant.

Plants can be cultivated by any known method in the art. Methods of cultivating plants are illustrated in, for example, "Moderu-shokubutsu-no-Jikken-Purotokoru For Ine · Shiroinunazuna : Saibo-kogaku Bessatsu-shokubutsu-saibo-kogaku sirizu 4; Ine-no-saibaiho [Experimental Protocol for Model Plants For Rice and *Arabidopsis thaliana*: Cellular Engineering, Special Issue, Plant Cellular Engineering Series 4; Rice Cultivating Methods]" (Kazutoshi Okuno) pp. 28-32, and "Arabidopushisu-no-saibaiho [Cultivating Methods for *Arabidopsis*]" (Yasuo Tanba) pp. 33-40 (Supervised by Ko Shimamoto and Kiyotaka Okada), which are not herein described in detail. For example, *Arabidopsis thaliana* can be cultivated by any of soil culture, rock wool culture, and water culture. After dissemination, flowering is first observed in about 4 weeks, if the plant is cultivated under constant light of a white color fluorescent lamp (about 6000 lux). After flowering, seeds are fully matured in about 16 days. Forty to fifty seeds are obtained from one pod. During 2 to 3 months from dissemination to death, about 10,000 seeds are obtained. The dormancy term of the seed is short. Fully-matured seeds after about one week drying are germinated 2 to 3 days after absorbing water. Note that if the seeds are subjected to cryogenic processing at 4°C for 2 to 4 days after water absorption and dissemination, the seeds are simultaneously germinated.

The expression analysis of the promoters of the present invention may be conducted using a gene analysis method using a DNA array. DNA arrays have been widely reviewed (Shujunsha Ed., Saibo-kogaku (Cellular Engineering), Special issue, "DNA-maikuro-arei-to-saisin-PCR-ho [DNA microarray and Up-to-date PCR Method"). Further, plant analysis using a DNA array has been recently used (Schenk PM et al. (2000) Proc. Natl. Acad. Sci. (USA) 97: 11655-11660). Hereinafter, a DNA array and a gene analysis method using the same will be briefly described.

"DNA array" refers to a device in which DNAs are arrayed and immobilized on a plate. DNA arrays are divided into DNA macroarrays, DNA microarrays, and the like according to the size of a plate or the density of DNA placed on the plate.

The border between macro and micro is not strictly determined. However, generally, "DNA macroarray" refers to a high density filter in which DNA is spotted on a membrane, while "DNA microarray" refers to a plate of glass, silicon, and the like which carries DNA on a surface thereof. A cDNA array, an oligoDNA array, and the like are used depending on the type of DNA placed thereon.

A certain high density oligoDNA array, in which a photolithography technique for production of semiconductor integrated circuits is applied and a plurality of oligoDNAs are simultaneously synthesized on a plate, is particularly called "DNA chip", an adaptation of the term "semiconductor chip". Examples of the DNA chip prepared by this method include GeneChip® (Affymetrix, CA), and the like (see Marshall A. et al., (1998) Nat. Biotechnol. 16: 27-31; and Ramsay G. et al., (1998) Nat. Biotechnol. 16 40-44). Preferably, GeneChip® may be used in gene analysis using a microarray according to the present invention. The DNA chip is defined as described above in the narrow sense, but may refer to all types of DNA arrays or DNA microarrays.

Thus, DNA microarrays are a device in which several thousands to several ten thousands or more gene DNAs are arrayed on a glass plate in high density. Therefore, it is made possible to analyze gene expression profiles or gene polymorphism at a genomic scale by hybridization of cDNA, cRNA or genomic DNA. With this technique, it has been made possible to analyze a signal transfer system and/or a transcription control pathway (Fambrough D. et al.,(1999), Cell 97, 727-741); the mechanism of tissue repair (Iyer VR. et al.,(1999), Science 283: 83-87); the action mechanism of medicaments (Marton MJ, (1999), Nat. Med. 4: 1293-1301); fluctuations in gene expression during development and differentiation processes, and the like; identify a gene group whose expression fluctuates according to pathologic conditions; find a novel gene involved in a signal transfer system or a transcription control; and the like. Further, as to gene polymorphism, it has been made possible to analyze a number of SNP with a single DNA microarray (Cargill M. et al., (1999), Nat. Genet. 22:231-238).

The principle of assays using a DNA microarray will be described. DNA microarrays are prepared by immobilizing a number of different DNA probes in high density on a solid-phase plate, such as a slide glass, whose surface is appropriately processed. Thereafter, labeled nucleic acids (targets) are subjected to hybridization under appropriate hybridization conditions, and a signal from each probe is detected by an automated detector. The resultant data is subjected to massive analysis by a computer. For example, in the case of gene monitoring, target cDNAs integrated with fluorescent labels by reverse transcription from mRNA are allowed to hybridize to oligoDNAs or cDNAs as a probe on a microarray, and are detected with a fluorescence image analyzer. In this case, T7 polymerase may be used to carry out other various signal amplification reactions, such as cRNA synthesis reactions or via enzymatic reactions.

Fodor et al. has developed a technique for synthesizing polymers on a plate using a combination of combinatorial chemistry and photolithography for semiconductor production (Fodor SP et al.,(1991) Science 251: 767-773). This is called the synthesized DNA chip. Photolithography allows extremely minute surface processing, thereby making it possible to produce a DNA microarray having a packing density of as high as 10 µm²/DNA sample. In this method, generally, about 25 to about 30 DNAs are synthesized on a glass plate.

Gene expression using a synthesized DNA chip was reported by Lockart et al. (Lockart DJ. et al., (1996) Nat. Biotechnol.: 14: 1675-1680). This method overcomes a drawback of the chip of this type in that the specificity is low since the length of synthesized DNA is short. This problem was solved by preparing perfect match (PM) oligonucleotide probes corresponding to from about 10 to about 20 regions and mismatch (MM) oligonucleotide probes having one base mutagenesis in the middle of the PM probes for the purpose of monitoring the expression of one gene. Here, the MM probes are used as an indicator for the specificity of hybridization. Based on the signal ratio between the PM probe and the MM probe, the level of gene expression may be determined. When the signal ratio between the PM probe and the MM probe is substantially 1:1, the result is called cross hybridization, which is not interpreted as a significant signal.

A so-called attached DNA microarray is prepared by attaching DNAs onto a slide glass, and fluorescence is detected (see also http://cmgm.stanford.edu/pbrown). In this method, no gigantic semiconductor production machine is required, and only a DNA array machine and a detector can be used to perform the assay in a laboratory. This method has the advantage that it is possible to select DNAs to be attached. A high density array can be obtained by spotting spots having a diameter of 100 µm at intervals of 100 µm, for example. It is theoretically possible to spot 2500 DNAs per cm2. Therefore, a usual slide glass (the effective area is about 4 cm²) can carry about 10,000 DNAs.

As a labeling method for synthesized DNA arrays, for example, double fluorescence labeling is used. In this method, two different mRNA samples are labeled by respective different fluorescent dyes. The two samples are subjected to competitive hybridization on the same microarray, and both fluorescences are measured. By comparing the fluorescences, a difference in gene expression is detected. Examples of the fluorescent dye include, but are not limited to, Cy5 and Cy3, which are most often used, and the like. The advantage of Cy3 and Cy5 is that the wavelengths of fluorescences do not overlap substantially. Double fluorescence labeling may be used to detect mutations or morphorisms in addition to differences in gene expression.

An array machine may be used for assays using a DNA array. In the array machine, basically, a pin tip or a slide holder is moved in directions along X, Y and Z axes in combination with a high-performance servo motor under the control of a computer so that DNA samples are transferred from a microtiter plate to the surface of a glass slide. The pin tip is processed into various shapes. For example, a DNA solution is retained in a cloven pen tip like a crow's bill and spotted onto a plurality of glass slides. After washing and drying cycles, a DNA sample is then placed on the glass slides. The above-described steps are repeated. In this case, in order to prevent contamination of the pin tip by a different sample, the pin tip has to be perfectly washed and dried. Examples of such an array machine include SPBIO2000 (Hitachi Software Engineering Co., Ltd.; single strike type), GMS417 Arrayer (Takara Shuzo Co., Ltd.; pin ring type), Gene Tip Stamping (Nippon Laser&Electronics Lab.; fountain pen type), and the like.

There are various DNA immobilizing methods for use in assays using a DNA array. Glass as a material for a plate has a small effective area for immobilization and electrical charge amount as compared to membranes, and therefore is given various coatings. In practice, Poly L-lysine coating (see Schena M. et al, (1995) Science 270: 467-470)), silane finishing (see Schena M. et al, (1996) Proc. Natl. Acad. Sci. (USA) 93: 10614-10619), or the like. Further, a commercially available precoated glass slide exclusive to DNA microarrays (e.g., polycarboimide glass (Nissin Spinning Co., Ltd.) and the like) may also be used. In the case of oligoDNA, a method of aminating a terminal of the DNA and crosslinking the DNA to silane-finished glass is available.

DNA microarrays may carry mainly cDNA fragments amplified by PCR. When the concentration of cDNA is insufficient, signals cannot be sufficiently detected in some cases. In such a case when a sufficient amount of cDNA fragments is not obtained by one PCR operation, PCR is repeated some times. The resultant overall PCR products may be purified and condensed at one time. A probe cDNA may generally carry a number of random cDNAs, but may carry a group of selected genes (e.g., the gene or promoter groups of the present invention) or candidate genes for gene expression changes obtained by RDA (representational differential analysis) according to the purpose of an experiment. It is preferable to avoid overlapping clones. Clones may be prepared from a stock cDNA library, or cDNA clones may be purchased.

In assays using a DNA array, a fluorescent signal indicating hybridization on the DNA microarray is detected by a fluorescence detector or the like. There are various conventional detectors available. For example, a research group at Stanford University has developed an original scanner which is a combination of a fluorescence microscope and a movable stage (see http://cmgm.stanford.edu/pbrown). A conventional fluorescence image analyzer for gels, such as FMBIO (Hitachi Software Engineering), Storm (Molecular Dynamics), and the like, can read a DNA microarray if the spots are not arrayed in a very high density. Examples of other available detectors include ScanArray 4000 and 5000 (GeneralScanning; scan type (confocal type)), GMS418 Array Scanner (Takara Shuzo; scan type (confocal type)), Gene Tip Scanner (Nippon Laser&Electronics Lab.; scan type (non-confocal type)), Gene Tac 2000 (Genomic Solutions; CCD camera type)), and the like.

The amount of data obtained from DNA microarrays is huge. Software for managing correspondences between clones and spots, analyzing data, and the like is important. As such software, software attached to each detection system is available (Ermolaeva O. et al.,(1998) Nat. Genet. 20:19-23). Further, an example of a database format is GATC (genetic analysis technology consortium) proposed by Affymetrix.

The promoter and functional plant of the present invention may also be analyzed by gene analysis using a differential display technique.

The differential display technique is a method for detecting or identifying a gene whose expression fluctuates. In this method, cDNA is prepared from each of at least two samples, and amplified by PCR using a set of any primers. Thereafter, a plurality of generated PCR products are separated by gel electrophoresis. After the electrophoresis pattern is produced, genes having fluctuating expression are cloned based on a relative signal strength change between each band.

Databases used in the present invention may include results derived from DNA chips. Further, the specificity of the promoters of the present invention may be analyzed and determined based on results of analysis of expression frequency using DNA chips.

### BEST MODE FOR CARRYING OUT THE INVENTION

In one aspect, the present invention provides a functional plant. Such a functional plant comprises at least one promoter promoting a specific level of expression and a gene operably linked to the promoter. The specificity of the promoter is determined based on the expression frequency of the gene including the promoter in a cDNA database in which the gene including the promoter is included. Such a method for analyzing expression frequency is well known in the art and described throughout herein.

In one embodiment, the genes to be used in the functional plant of the present invention are not expressed in fruits. In another embodiment, the genes are specifically expressed in leaves. In another embodiment, the genes are specifically expressed in callus.

The functional plants of the present invention may be monocotyledonous or dicotyledonous. In one embodiment, the functional plant of the present invention may be monocotyledonous. In one preferable embodiment, the functional plant of the present invention may be cereal plant (for example, wheat, maize, rice, barley, sorghum and the like). More preferably, the functional plant of the present invention is rice. In another preferable embodiment, the functional plant may be crops for food or other taste material such as tobacco, green pepper, aubergine, melon, tomato, sweet potato, cabbage, green onion, broccoli, carrot, cucumber, oranges, Chinese cabbage, lettuce, peach, potato, and apple. In another embodiment, the functional plant may be plants for ornamentation such as plants in which flowers are appreciated such as petunia, carnation, chrysanthemum, Turkish balloon flower, plants in which trees are appreciated such as pines, or the like.

In other embodiments, genes used in the present invention may be disease resistance genes or insect resistance genes such as difensin, somatin, chitinase, Bt, oryzacystatin, or the like.

In a preferable embodiment, the genes used in the present invention may be a gene selected from the group consisting of a vitamin synthesizing gene, a carbohydrate synthesizing gene, a lipid synthesizing gene, a polyketide synthesizing gene, a photosynthesizing system gene, a functional component synthesizing gene and a transcription factor.

In another aspect, the present invention is related to a promoter promoting a specific level of expression. The specificity of the promoter is determined based on the expression frequency of the gene including the promoter in a cDNA database in which the gene including the promoter is included.

In one embodiment, the promoter of the present invention does not drive expression of a gene in fruits. In another embodiment, the promoter of the invention specifically drives a specific level of expression of a gene in leaves. In another embodiment, the promoter of the present invention drives a specific level of expression of a gene only in an available site. In still another embodiment, the promoter of the present invention drives a specific level of expression of a gene in a stem. In a still further embodiment, the promoter of the present invention drives a specific level of expression in flowers. In still another embodiment, the promoter of the present invention drives a specific level of expression of a gene in fruits. In still different embodiment, the promoter of the present invention drives a specific level of expression of a gene in seeds.

In another aspect, the present invention provides an expression cassette comprising a promoter promoting a specific level of gene expression. The specificity of the promoter is determined based on the expression frequency of the gene including the promoter in a cDNA database in which the gene including the promoter is included.

In one embodiment, the expression cassette of the present invention does not drive specific expression of a gene in fruits. In another embodiment, the expression cassette of the present invention drives a specific level of expression of a gene in leaves. In another embodiment, the expression cassette of the present invention drives a specific level of expression of a gene only in an available site. In still another embodiment, the expression cassette of the present invention drives a specific level of expression of a gene in a stem. In a still further embodiment, the expression cassette of the present invention drives a specific level of expression in flowers. In still another embodiment, the expression cassette of the present invention drives a specific level of expression of a gene in fruits. In still a different embodiment, the expression cassette of the present invention drives a specific level of expression of a gene in seeds.

In another aspect, the present invention provides an available site of a plant which is obtained from a plant comprising at least one promoter promoting a specific level of expression and a gene operably linked to the promoter. The specificity of the promoter is determined based on the expression frequency of the gene including the promoter in a cDNA database in which the gene including the promoter is included. Preferably, the available site may be leaves or fruits. In a different preferable embodiment, the promoter used therein does not drive expression in an available site. In this case, the genes may be disease resistance genes or insect resistance genes such as difensin, somatin, chitinase, Bt, oryzacystatin, or the like. In one embodiment, the promoter drives specific gene expression in an available site. In this case, the genes may be a gene selected from the group consisting of a vitamin synthesizing gene, a carbohydrate synthesizing gene, a lipid synthesizing gene, a polyketide synthesizing gene, a photosynthesizing system gene, a functional component synthesizing gene and a transcription factor.

In another aspect, a method for producing a plant specifically expressing a desired gene product. The present methods comprises the steps of: determining the specificity of a promoter wherein the specificity of the promoter is determined based on the expression frequency of the gene including the promoter in a cDNA database in which the gene including the promoter is included; producing a linked mixture by operably linking the promoter and a nucleic acid molecule encoding the desired gene product; introducing the linked mixture into a plant; and growing the plant.

The present invention also provides a method for producing a desired gene product. The methods comprise the steps of: determining the specificity of a promoter wherein the specificity of the promoter is determined based on the expression frequency of the gene including the promoter in a cDNA database in which the gene including the promoter is included; producing a linked mixture by operably linking the promoter and a nucleic acid molecule encoding the desired gene product; introducing the linked mixture into a plant; growing the plant; and collecting the desired gene product in the plant.

In an embodiment, the specificity of the promoter may be determined based on the expression frequency of the gene including the promoter in a cDNA database in which the gene including the promoter is included.

In an aspect, the present invention provides a gene product obtainable by a method of the present invention. Such a gene product may be a pharmaceutical product such as antibiotics, polypeptides, proteins or the like. The second metabolic product of a gene may be obtained by a method of the present invention.

Hereinafter, the present invention will be described by way of examples. Examples described below are provided only for illustrative purposes.
Accordingly, the scope of the present invention is not limited by the above-described embodiments or the examples below except as by the appended claims.

### EXAMPLES

### (Example 1: Identification of specific promoters using a cDNA library)

### (RNA extraction of DNA from rice tissues)

RNA was extracted from the following rice tissues: benzyl adenine treated callus, gibberellin treated callus, heat shock treated callus, callus in growing phase, young root, young green leaves, young etiolated leaves, and ear of blooming period. In brief, triturated plant tissues were treated with guanidine thiocyanate and phenol to degrade proteins, total RNA was collected together with aqueous layer. As mRNA of an eukaryotic organism has poly A sequence at 3' terminus, such mRNA can be specifically isolated by adsorption of the treated solution with a carrier with Oligo-dT immobilized thereto and elution.

### (Construction,of cDNA library from extracted RNA)

The extracted mRNA was used to produce complementary DNA chain (1^{st} strand) using reverse transcriptase and RnaseH and DNA polyperase was used to produce 2^{nd} strand, resulting in the production of a cDNA library.

### (Analysis of a cDNA library)

The resultant cDNA library was compared with known sequences registered in DDBJ, and the expression frequency was summarized according to identical or similar genes. First, clones having high homology of more than 90% over the entire sequence was extracted from the result of the homology search. Second, the resultant clones were compared to each other, and analyzed for the presence or absence of the difference in the base level at a particular site in the sequences. In the case where there are apparent at least two base bias at a specific site, it was determined that there are cognate different genes. In the case where there is no bias, it is determined that there was just a reading error by the automatic base sequencing apparatus. Hereinbelow, an exemplary result in which there are identical examples, is shown in Figure 1 below. The result is also shown below.

**TABLE 1**

| ID | #of MEM | CB | CG | CH | CK | RA | SS | ST | EB | EC | OPT .S | ACC | TITLE |
|---|---|---|---|---|---|---|---|---|---|---|---|---|---|
| EC0884_1A | 207 | 0 | 0 | 0 | 0 | 0 | 0 | 0 | 0 | 207 | 580 | S31078 | seed allergen RA5 |
| CH3160_1A | 148 | 2 | 2 | 142 | 0 | 0 | 1 | 1 | 0 | 0 | 557 | S20874 | Heat shock protein |
| ST4576_2P | 102 | 3 | 2 | 5 | 0 | 18 | 15 | 53 | 3 | 3 | 540 | S41194 | transmembrane protein |
| CH0671_1A | 99 | 12 | 16 | 34 | 11 | 3 | 3 | 9 | 3 | 3 | 723 | S25541 | Heat shock protein 82 (HSP82) |
| SS0238_1B | 67 | 0 | 0 | 0 | 0 | 0 | 52 | 11 | 0 | 4 | 121 | A25707 | U1 snRNP 70K protein |
| CB0859_1A | 65 | 34 | 16 | 7 | 8 | 0 | 0 | 0 | 0 | 0 | 112 | S34045 | protamine |
| SS0881_1B | 63 | 0 | 0 | 0 | 0 | 0 | 58 | 0 | 0 | 5 | 628 | S29048 | fructose-bisphosphate aldolase |
| CD0073_2A | 55 | 1 | 10 | 10 | 31 | 0 | 1 | 1 | 0 | 0 | 133 | A40973 | Spermatid-specitic protein T1 |
| CG0060_1A | 36 | 0 | 31 | 1 | 4 | 0 | 0 | 0 | 0 | 0 | | | |
| EB0082_2A | 34 | 0 | 0 | 0 | 0 | 0 | 31 | 0 | 2 | 1 | 429 | A48527 | photosystem I protein psaK precursor |
| EB0319_1A | 27 | 0 | 0 | 0 | 0 | 0 | 23 | 0 | 3 | 1 | 371 | S04125 | Chlorophyl I a/b-binding protein type III precursor |
| RA1583_1B | 27 | 4 | 3 | 1 | 0 | 1 | 12 | 5 | 0 | 0 | 372 | S16636 | Auxin-induced protein (clone pCNT107) |
| SS5153_1A | 25 | 0 | 0 | 0 | 0 | 0 | 23 | 0 | 2 | 0 | 480 | S33443 | CP29 protein |
| SS0410_1A | 21 | 0 | 0 | 0 | 0 | 0 | 21 | 0 | 0 | 0 | | | |
| EB0105_1A | 21 | 0 | 0 | 0 | 0 | 0 | 20 | 0 | 1 | 0 | 451 | S21386 | Chlorophyl I a/b-binding protein CP29 precursor |
| CD0049_1A | 20 | 0 | 2 | 1 | 1 | 0 | 0 | 15 | 0 | 0 | 621 | S37043 | salT protein |
| RA0087_1B | 20 | 0 | 0 | 0 | 0 | 1 | 0 | 19 | 0 | 0 | 201 | TIRZBR | trypsin inhibitor (Bowman-Birk) |

In the Table, CB refers to benzyl adenine treated callus. CG refers to gibberellin treated callus. CH refers to heat shock treated callus. CK refers to callus in growing phase. RA refers to young root. SS refers to young green leaves. ST refers to young etiolated leaves. EB refers to ear in blooming period. EC refers to ear in early maturation period.

Based on the table above, it was possible to select promoters showing a desired expression pattern. In this example, for example, dedifferentiation state (callus) specific promoters, leave-specific promoters, constitutive promoters were selected.

Dedifferentiation state (callus) specific promoters were obtained from promoter regions of genes only showing expression in at least one site selected from the group consisting of CB, CG, CH and CK, or showing greater expression in at least one site selected from the group consisting of CB, CG, CH and CK than the other sites. Here, a clone belonging to ID CG0060_1 was used in the following Examples. The sequence thereof is shown in SEQ ID NO: 1.

Leave-specific promoters were obtained from promoter regions of genes only showing expression in at least one site selected from the group consisting of SS and ST, or showing greater expression in at least one site selected from the group consisting of SS and ST than the other sites. Here, a clone belonging to ID EB0082_2 was used in the following Examples. The sequence thereof is shown in SEQ ID NO: 2.

Constitutive promoters were selected from promoter regions of a gene showing substantially the same expression over entire sites. Here, a clone belonging to ID RA1581_1 was used in the following Examples. The sequence thereof is shown in SEQ ID NO: 3.

### (Example 2: Determination of the specificity of the selected promoters)

In this example, each of the selected clones in Example 1 was determined as to whether or not each has a specificity of interest. Specific protocols are described below.

### (Base sequencing of the selected clones)

First, it was determined whether or not the selected clones are identical to the deduced sequence. Sequencing was conducted by the dideoxy dye termination method, which is well known to those skilled in the art. Briefly, the DNA chain to be sequenced is made single stranded by heat denaturation, and primers were annealed to the upstream of the portions to be sequenced. In the presence of dideoxy ribonucleotide triphosphate with different fluorophore added depending on the base and deoxynucleotide triphosphate, DNA synthesis reacton by DNA polymerase is conducted. When dideoxy ribonucleotide triphosphate was incorporated, the DNA synthesis elongation was stopped. The resultant product was separated according to molecular weight by electrophoresis of acrylamide gel, and reading the fluorophore by an apparatus to determine the base sequences.

Further, the determined sequences were compared with the sequence registered at DDBJ to confirm that the above selected clones have the sequence of interest.

### (Example 3: Obtaining promoter regions)

In this example, promoter regions were obtained by using clones which were confirmed to have the sequence of interest. Brief protocols are described below.

First, primers were designed based on the data registered at DDBJ. A kit for isolating upstream of a known sequence using PCR based technology which is commercially available (GeneomWalker; Clontech, USA) was used to clone and obtain the promoter regions of genes of interest. The scheme is shown in Figure 2.

These fragments were from 0.6 to 2.5 kb in length.

### (Example 4: Production of a functional plant using callus specific promoters)

### (Production of expression vectors using Green Fluorescence Protein)

The Green Fluorescence Protein gene and promoter region derived from clone of CG0060_1 (SEQ ID NO: 1) which is expected to be a promoter driving a specific level of expression in callus, were used to produce an expression vector. Specific protocols are described below.

Rice genome DNA was totally digested using the restriction enzyme EcoRV and adapter primers of GenomeWalker (SEQ ID NO: 4) were added at both termini of the resultant DNA fragments. PCR reaction using the primer pair designed based on sequence inside the adapter primer and known sequences, were conducted to obtain primer regions.

Restriction enzyme recognition sequences were added to both termini of the obtained fragments for ease of use. The 5' terminus primer (SEQ ID NO: 5) was designed to comprise a portion of the adapter primer and a restriction enzyme recognition sequence. The 3' terminus primer (SEQ ID NO: 6) was designed to comprise the sequence just prior to the translation initiation site, and a restriction enzyme recognition sequence. These primers were used to conduct PCR reaction to produce a promoter cassette (SEQ ID NO: 7) with restriction enzyme recognition sequences added to both termini.

A DNA fragment having a construct in which a Green Fluorescence Protein gene is promoted by the promoter of the present invention and has a transcriptional stop signal sequence derived from Agrobacterium nopaline synthase gene which are inserted between the Hind III and EcoRI sites of the multicloning site designed within a binary vector pTN1 to produce a binary vector to be used.

### (Introduction of the expression vector into rice)

Rice was transformed with the produced vector. The specific procedure is as follows:

The produced vector was introduced into an Agrobacterium by electorporation to result in a transformed *Agrobacterium*. A rice seed, which had been precultured, was infected with the *Agrobactrium* by coculturing the seed and the transformed *Agrobacterium* for three days. The seed used for the transformed rice was fully matured, disinfected with ethanol and sodium hypochlorite solution, and cultured on a medium containing 2,4-D for five days.

### (Results)

As a result, a rice cell transformed with the promoter was obtained.

### (Selection of infected cells)

The gene nptII encoded by the pTN vector in the transformed rice cell renders the cell resistant to geneticin. Therefore, only transformed rice cells were selected by culturing the produced cells in N6 medium containing geneticin.

### (Redifferentiation of transformed rice)

The transformed rice cell was then redifferentiated. The specific procedure is as follows:

The transformed rice cells were cultured on a medium containing cytokinin for inducing redifferentiation, and then grown on a medium without hormone until the cells were ready for culture on soil, and thereafter the culture was naturalized.

### (Results)

As a result, each of the plant tissues were obtained.

### (Identification of specific expression)

The resultant callus and plant body were separated into each region (root, leaves, stem, albumen, and germ) and expression properties were determined.

### (Results)

The results obtained are shown in Figures 3 to 5. As shown in the figures, a promoter derived from CG0060_1 was demonstrated not to drive specific expression in root.

Further, the promoter was demonstrated to drive specific expression in callus. The expression driven by the promoter was demonstrated to be less than detection limitation in leaves and rice grain.

### (Example 5: Production of a functional plant using a leaf-specific promoter)

### (Production of expression vectors using Green Fluorescence Protein)

The Green Fluorescence Protein gene and promoter region derived from clone of EB0082_2 (SEQ ID NO: 2) which is expected to be a promoter driving specific expression in leaves, were used to produce an expression vector.

The rice genome DNA was totally digested using the restriction enzyme DraI and adapter primers of GenomeWalker (SEQ ID NO: 4) were added at both terminus of the resultant DNA fragments. PCR reaction using the primer pair designed based on sequence inside the adapter primer and known sequences, were conducted to obtain primer regions.

Restriction enzyme recognition sequences were added to both terminis of the obtained fragments for the ease of use. The 5' terminus primer (SEQ ID NO: 8) was designed to comprise a portion of the adapter primer and a restriction enzyme recognition sequence. The 3' terminus primer (SEQ ID NO: 9) was designed to comprise the sequence just prior to the translation initiation site, and a restriction enzyme recognition sequence. These primers were used to conduct the PCR reaction to produce a promoter cassette (SEQ ID NO: 10) with restriction enzyme recognition sequences added to both termini.

### (Introduction of the expression vector into rice)

Rice was transformed with the produced vector. In brief, a DNA fragment having a construct in which a Green Fluorescence Protein gene is promoted by the promoter of the present invention and has a transcriptional stop signal sequence derived from *Agrobacterium* nopaline synthase gene which are inserted between the Hind III and EcoRI sites of the multicloning site designed within a binary vector pTN1 to produce a binary vector to be used.

### (Results)

As a result, a rice cell transformed with the promoter was obtained.

### (Production of callus from transformed rice)

Next, a callus was produced from the transformed rice. The specific procedure is the same as in Example 7.

### (Results)

As a result, plant tissues were obtained.

### (Production of redifferentiated transformed rice)

Then, the transformed rice was redifferentiated. The specific procedures are the same as in Example 7.

### (Results)

As a result, a plant body was obtained.

### (Identification of specific expression)

The resultant callus and plant body were separated into each region (root, leaves, stem, albumen, and germ) and expression properties were determined.

### (Results)

The results obtained are shown in Figures 3 to 5. As shown in the figures, a promoter derived from EB0082_2 was demonstrated not to drive specific expression in root (Figure 3).

Further, the promoter was demonstrated to drive a specific level of expression in leaves (Figure 4). The expression driven by the promoter was demonstrated to be less than detection limitation in rice grain (Figure 5).

### (Example 6: Production of functional plants using a promoter not promoting albumen)

### (Production of expression vectors using Green Fluorescence Protein)

The Green Fluorescence Protein gene and promoter region derived from clone of RA1583_1 (SEQ ID NO: 3) which is expected to be a promoter driving no specific expression in albumen, were used to produce an expression vector.

Rice genome DNA was totally digested using restriction enzyme PvuII and adapter primers of GenomeWalker (SEQ ID NO: 4) were added at both termini of the resultant DNA fragments. PCR reaction using the primer pair designed based on sequence inside the adapter primer and known sequences, were conducted to obtain primer regions.

Restriction enzyme recognition sequences were added to both termini of the obtained fragments for the ease of use. The 5' terminus primer (SEQ ID NO: 5) was designed to comprise a portion of the adapter primer and a restriction enzyme recognition sequence. The 3' terminus primer (SEQ ID NO: 11) was designed to comprise the sequence of just prior to the translation initiation site, and a restriction enzyme recognition sequence. These primers were used to conduct the PCR reaction to produce a promoter cassette (SEQ ID NO: 12) with restriction enzyme recognition sequences added to both termini.

### (Introduction of the expression vector into rice)

Rice was transformed with the produced vector. In brief, a DNA fragment having a construct in which a Green Fluorescence Protein gene is promoted by the promoter of the present invention and has a transcriptional stop signal sequence derived from Agrobacterium nopaline synthase gene which are inserted between the Hind III and EcoRI sites of the multicloning site designed within a binary vector pTN1 to produce a binary vector to be used.

### (Results)

As a result, a rice cell transformed with the promoter was obtained.

### (Selection of the infected cells)

Then, infected rice was selected. The specific procedure therefore is the same as in Example 7.

### (Redifferentiation of transformed rice)

Next, the transformed rice was redifferentiated. The specific procedure is the same as in Example 7.

### (Results)

As a result, each of the plant tissues was obtained.

### (Identification of specific expression)

The resultant callus and plant body were separated into each region (root, leaves, stem, albumen, and germ) and expression properties were determined.

### (Results)

The results obtained are shown in Figures 3 to 5. As shown in the figures, a promoter derived from RA1583_1 was demonstrated to drive specific expression in leaves (Figure 4). No specific expression in root was driven by this promoter region.

It was demonstrated that albumen of rice grain as an available site was detected to have specific expression.

### (Example 7: Demonstration of utility of promoter regions of clones driving callus specific expression)

Next, a promoter region of a clone derived from CG0060_1 which is expected to be a promoter driving specific expression in callus was tested for its utility of the expression strength of the promoter. The specific procedure is described as follows.

### (Linkage of the promoter region and a reporter gene)

Next, an expression vector was used to confirm the utility of the expression strength of the promoter region by linking the promoter region of the clone and HPT, which is an antibiotic resistant gene, in the vector. The procedure therefore is described as follows:

A binary vector pTH1 having a construct driving the HPT gene by the CaMV 35S promoter was processed to remove the CaMV 35S promoter region, and to insert the promoter of the present invention in the place thereof to produce a binary vector having a construct driving the HPT gene driven by the promoter of the present invention. A method similar to the ultrafast transformation method of monocotyledonous plant (Japanese Patent No.: 3141084) was used for the introduction of a gene into rice cells.

### (Results)

The results are shown in Figures 6 and 7.

As shown in the electrophoresis diagram in Figure 6, the rice callus with the inserted HPT gene was produced and grown in the present Example. Further, as shown in Figure 7, the transformed callus produced by the vector of the present Example has been rendered resistant to hygromycin. As a positive control, the results of pTH in which the CaMV 35S promoter is present and which drives the expression of the HPT gene, are also shown.

The vector with the promoter of the present invention used in the Example introduced was demonstrated to have sufficient expression strength to render the cell resistant to hygromycin. A wild-type used as a control has not been rendered hygromycin resistant (Figure 7). Accordingly, a clone derived from CG0060_1 was demonstrated to have callus specific promoter region.

### (Example 8: Demonstration of utility of a promoter region not driving expression in albumen)

Next, a promoter region of a clone derived from RA1583_1 which is expected to be a promoter not driving specific expression in albumen was tested for its utility of the expression strength of the promoter. The specific procedure is described as follows.

### (Linkage of the promoter region and a reporter gene)

Next, an expression vector was used to confirm the utility of the expression strength of the promoter region by linking the promoter region of the clone and an antibiotic resistant gene in the vector. The procedure therefore is the same as in Example 7.

### (Results)

The results are shown in Figures 6 and 7.

As shown in the electrophoresis diagram in Figure 6, the rice callus with the inserted hygromycin phosphoryl transferase gene was produced and grown in the present Example. Further, as shown in Figure 7, the transformed callus produced by the vector of the present Example has been rendered resistant to hygromycin.

The vector with the promoter of the present invention used in the Example was demonstrated to have sufficient expression strength to render the cell resistant to hygromycin. A wild-type used as a control has not been renderedhygromycin resistant. Accordingly, a clone derived from RA1583_1 was demonstrated to have sufficient expression strength for rendering hygromycin resistance.

### (Comparative Example)

For comparison, the same experiments have been conducted except for the use of the promoter derived from CaMV 35S. Similarly, the comparative promoter also rendered the resultant plant resistance to hygromycin.

### (Conclusion)

As shown above, it was demonstrated that a functional plant using a promoter having specificity determined based on the expression frequency of genes containing promoter in a cDNA database.

It is an epoch-making technology in which such functional plants have been able to be designed and produced, which has not been permissible according to prior art technology, and the utility in industry should be noted.

### (Effects of the present invention)

The present invention provides a functional plant in which gene expression pattern is altered so that specific expression is driven or not driven in a desired site.

### INDUSTRIAL APPLICABILITY

According to the method of the present invention for producing a functional plant, a system for expressing a desired gene in a desired site of a plant in an arbitrary manner, has been provided. According to the present method, a functional plant in which a gene product, is or is not expressed in a desired site, is provided. It should be noted to be industrially applicable that such a functional plant is arbitrarily provided.

## Claims

1. A plant comprising at least one promoter promoting a specific level of expression and a gene operably linked to the promoter, wherein the specificity of the promoter is determined based on the expression frequency of the gene including the promoter in a cDNA database in which the gene including the promoter is included.

2. The plant according to claim 1 wherein the gene is constitutively expressed.

3. The plant according to claim 1 wherein the gene is specifically expressed in leaves.

4. The plant according to claim 1 wherein the gene is specifically expressed in callus.

5. The plant according to claim 1 wherein the plant is rice.

6. The plant according to claim 1 wherein the gene is a disease resistance gene or an insect resistance gene.

7. The plant according to claim 1 wherein the gene is a gene selected from the group consisting of a vitamin synthesizing gene, a carbohydrate synthesizing gene, a lipid synthesizing gene, a polyketide synthesizing gene, a photosynthesizing system gene, a functional component synthesizing gene and a transcription factor.

8. A promoter promoting a specific level of expression, wherein the specificity of the promoter is determined based on the expression frequency of the gene including the promoter in a cDNA database in which the gene including the promoter is included.

9. The promoter according to claim 8 which does not drive the expression of a gene in fruit.

10. The promoter according to claim 8 which specifically drives the expression of a gene in leaves.

11. The promoter according to claim 8 which specifically drives the expression of a gene in callus.

12. An expression cassette comprising a promoter wherein the specificity of the promoter is determined based on the expression frequency of the gene including the promoter in a cDNA database in which the gene including the promoter is included.

13. The expression cassette according to claim 12 which promotes the constitutive expression of a gene.

14. The expression cassette according to claim 12 which specifically drives the expression of a gene in leaves.

15. The expression cassette according to claim 12 which specifically drives the specific expression of a gene in callus.

16. An available site of a plant which is obtained from a plant comprising at least one promoter promoting a specific expression and a gene operably linked to the promoter, wherein the specificity of the promoter is determined based on the expression frequency of the gene including the promoter in a cDNA database in which the gene including the promoter is included.

17. The available site according to claim 16 which is a leaf, a stem or a fruit.

18. A method for producing a desired gene product, comprising the steps of:
determining the specificity of a promoter wherein the specificity of the promoter is determined based on the expression frequency of the gene including the promoter in a cDNA database in which the gene including the promoter is included;
producing a linked mixture by operably linking the promoter and a nucleic acid molecule encoding the desired gene product;
introducing the linked mixture into a plant;
growing the plant; and
collecting the desired gene product in the plant.

19. A method for producing a plant specifically expressing a desired gene product, comprising the steps of:
determining the specificity of a promoter wherein the specificity of the promoter is determined based on the expression frequency of the gene including the promoter in a cDNA database in which the gene including the promoter is included;
producing a linked mixture by operably linking the promoter and a nucleic acid molecule encoding the desired gene product;
introducing the linked mixture into a plant; and
growing the plant.

20. The method according to claim 19 wherein the specificity of the promoter is determined by the analysis of a DNA chip.

21. A gene product which is obtained from the plant obtained by the method according to claim 19.
